(19) Europäisches Patentamt
European Patent Office
Office européen des brevets

(11) Veröffentlichungsnummer: **0 280 186 B1**

# (12) EUROPÄISCHE PATENTSCHRIFT

(45) Veröffentlichungstag der Patentschrift: **06.05.92**

(51) Int. Cl.$^5$: **A61K 7/13**

(21) Anmeldenummer: **88102335.2**

(22) Anmeldetag: **18.02.88**

## (54) Haarfärbemittel mit direktziehenden Nitrodiphenylamin-Derivaten.

(30) Priorität: **26.02.87 DE 3706223**

(43) Veröffentlichungstag der Anmeldung:
**31.08.88 Patentblatt 88/35**

(45) Bekanntmachung des Hinweises auf die Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
**EP-A- 0 010 244**
**EP-A- 0 220 614**

**Chemical Abstracts, Band 97, Nr. 4, 26. Juli 1982, Colombus, Ohio, USA; M. Muszynski; "Anthraquinone derivative acid dyes"**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Rose, David, Dr.**
**Holbeinweg 7**
**W-4010 Hilden(DE)**
Erfinder: **Lieske, Edgar**
**Hunsrückenstrasse 40**
**W-4000 Düsseldorf(DE)**
Erfinder: **Maak, Nobert, Dr.**
**Im Jagdfeld 41 a**
**W-4040 Neuss(DE)**

EP 0 280 186 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Haarfärbemittel mit einem Gehalt an direktziehenden Haarfarbstoffen. Solche Haarfärbemittel enthalten direktziehende Haarfarbstoffe in einem kosmetischen Träger. Oft enthalten solche Haarfärbemittel zur Erzielung bestimmter Nuancen zusätzlich Oxidationsfarbstoffvorprodukte. Als kosmetische Träger für die direktziehenden Haarfarbstoffe und gegebenenfalls Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, indophenolen, Triphenylmethanfarbstoffen, kationischen Azofarbstoffen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Unter den direktziehenden Nitrobenzolderivaten spielen die Nitroaniline und deren Derivate eine besondere Rolle, da einige dieser Farbstoffe intensive Färbungen von guter Lichtechtheit ausbilden. Ein Nachteil der bekannten direktziehenden Nitroanilin-Farbstoffe ist jedoch einerseits eine begrenzte Wasserlöslichkeit, die zu Problemen bei der Formulierung der Haarfärbemittel führt, andererseits die unbefriedigende Waschechtheit, d. h. daß die Haarfärbungen nach mehrmaligem Haarwaschen stark ausbluten.

Weiterhin ist es wünschenswert, um modische Haarfärbungen zu erhalten, auch rote Farbtöne durch direktziehende Farbstoffe zu erzeugen. Dafär werden gewöhnlich 2-Nitro-p-phenylendiamin und dessen amino-substituierte Derivate verwendet. Leider sind diese Stoffe in Wasser wenig löslich bzw. schwer dispergierbar. Dies führt leicht zu ungleichmäßigen oder zu schwachen Haarfärbungen. Insbesondere bei Färbezubereitungen mit hoher Farbstoffkonzentration kommt es leicht vor, daß Farbstoffe auskristallisieren und nicht auf das zu färbende Haar aufziehen. Es besteht daher ein dringendes Bedürfnis an direktziehenden Haarfarbstoffen mit verbesserter Wasserlöslichkeit.

Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Es wurde gefunden, daß die gestellten Anforderungen in hohem Maße erfüllt werden durch Haarfärbemittel mit einem Gehalt an direktziehenden Haarfarbstoffen in einem wäßrigen kosmetischen Träger, dadurch gekennzeichnet, daß als direktziehende Haarfarbstoffe eine oder mehrere Verbindungen der Formel (I)

(I) 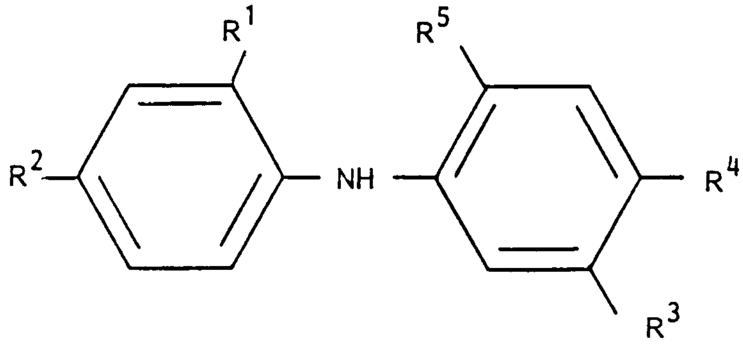

in der eine der Gruppen $R^1$ und $R^2$ eine Nitrogruppe und die andere eine $-SO_3$ H-Gruppe und eine der Gruppen $R^3$ und $R^4$ eine Gruppe $-NR^6R^7$ ist, worin $R^6$ und $R^7$ Wasserstoff, Alkylgruppen mit 1 bis 4 C-Atomen oder Hydroxyalkylgruppen mit 2 bis 4 C-Atomen sind, und die andere sowie die Gruppe $R^5$ Wasserstoff, Chlor oder Alkylgruppen mit 1 bis 4 C-Atomen sind, oder deren wasserlösliche Salze enthalten sind.

Unter den Verbindungen der Formel (1) sind wegen der leichten technischen Zugänglichkeit besonders solche bevorzugt, in welchen $R^3$ Wasserstoff, $R^4$ eine Gruppe $-NR^6R^7$ und $R^5$ Wasserstoff, Chlor oder eine Methylgruppe ist.

Die Nitrodiphenylaminderivate der Formel (1) erzeugen auf dem Haar tiefgelbe bis rotbraune Farbtöne von hoher Intensität, Licht-und Waschechtheit.

Die Herstellung der Nitrodiphenylaminderivate erfolgt allgemein durch Umsetzung von 4-Chlor-3-nitrobenzolsulfonsäure-Alkalisalz oder 2-Chlor-5-nitrobenzolsulfonsäure-Alkalisalz mit einem Paraphenylendiamin der Formel (II)

(II)

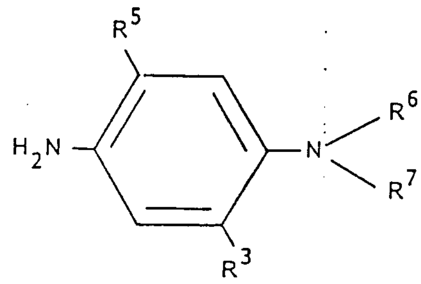

unter Abspaltung von HCl in Gegenwart einer Base, z.B. eines Alkalicarbonats. Dabei haben in Formel (II) die Gruppen $R^3$, $R^5$, $R^6$ und $R^7$ die gleiche Bedeutung wie für Formel (I) angegeben.

Ein geeignetes Verfahren zur Herstellung von 4-Nitro-4'-amino-diphenylamin-2-sulfonsäure und 2-Nitro-4'-amino-diphenylamin-4-sulfonsäure ist aus EP-A-10 244 bekannt. Die Verbindungen werden dort als Vorprodukte zur Herstellung von Farbstoffen beschrieben.

Unter den wasserlöslichen Salzen sind in erster Linie die Salze starker Basen wie etwa die Alkalisalze, z.B. das Natrium- oder Kaliumsalz oder Ammoniumsalze, Alkanolammoniumsalze mit 2 bis 4 C-Atomen in der Alkanolgruppe wie z.B. das Monoethanolammoniumsalz, das Triethanolammoniumsalz oder das Isopropanolammoniumsalz zu verstehen. Die erfindungsgemäßen Haarfärbemittel können die direktziehenden Nitrodiphenylaminderivate der allgemeinen Formel 1 allein oder in Kombination mit bekannten direktziehenden Farbstoffen, z. B. mit anderen Nitrobenzolderivaten, Anthrachinonfarbstoffen, Triphenylmethan- oder Azofarbstoffen enthalten. Darüber hinaus eignen sich die direktziehenden Farbstoffe der allgemeinen Formel 1 wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidantionshaarfärbemitteln. Oxidationshaarfärbemittelenthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder aminogruppe, Diaminopyridinderivate, heterocyclische Hydrazone, 4-Amino-pyrazolonderivate und 2,4,5,6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Oleinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl-oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin. Die Bestandteile der kosmetischen Träger werden zur Herstellung der

erfindungsgemäßen Haarfärbemittel in für diese Zwecke üblichen Mengen eingesetz; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den erfindungsgemäßen Haarfärbemitteln werden die direktziehenden Haarfarbstoffe der allgemeinen Formel 1 einer Menge von 0,01 bis 5,0 Gew.-%, bevorzugt von 0,1 bis 2 Gew.-%, bezogen auf das gesamte Haarfärbemittel, eingesetzt. Daneben können bekannte Oxidationshaarfärbemittelvorprodukte (Entwickler- und Kupplersubstanzen) in einer Menge von 0,01 bis 5, vorzugsweise von 1 bis 3 Gew.-% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder akalischen Milieu erfolgen. Bevorzugt ist die Anwendung der Haarfärbemittel in einem pH-Bereich von 8 bis 10. Die Anwendungstemperaturen können in eine Bereich zwischen 15 °C und 40 °C liegen. Nach einer Einwirkungszeit von ca. 30 Minuten wird das Haarfärbermittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponiern erzielen. Die nachfolgenden Beispiele sollen den Erfindungsgegenstand näher erläutern, ohne ihn jedoch hierauf zu beschränken.

## Beispiele

### Herstellungsbeispiele

1. 2-Nitro-4'-N,N-bis-(2-hydroxyethyl)-aminodiphenylamin-4-sulfonsäure-Natriumsalz

Eine Mischung aus (A) 6,5 g (0,025 Mol) 4-Clor-3-nitro-benzolsulfonsäure-Na-Salz und (B) 4,9 g (0,025 Mol) N,N-bis(2-hydroxyethyl)-p-phenylendiamin, 2,54 g (0,027 Mol) Natriumcarbonat und 10 ml Wasser wurde in einem Autoklaven 7 Stunden auf 120 °C erhitzt. Nach dem Abkühlen wurde die Lösung zur Trokkene eingeengt. Der Rückstand wurde aus einem Ethanol-Wasser-Gemisch umkristallisiert.
Rotbraune Kristalle, D. C. (Silikagel 60F-254/Merk)
Laufmittel : n-Butanol/Essigsäure/Wasser = 8:2:1; Rf = 0,42

2. 2-Nitro-2'-methyl-4'-aminodiphenylamin-4-sulfonsäure-Natriumsalz

Die Herstellung erfolgte analog Beispiel 1 ausgehend von
(A) 4-Chlor-3-nitrobenzolsulfonsäure-Na-Salz und
(B) p-Toluylendiamin
Rostrotes Pulver, D. C. (Silikagel 60F-254/Merck)
Laufmittel : n-Butanol/Essigsäure/$H_2O$ = 8:2:1; Rf = 0,43

3. 2-Nitro-2'-Chlor-4'-aminodiphenylamin-4-sulfonsäure-Natriumsalz

Herstellung analog Beispiel 1 ausgehend von
(A) 4-Chlor-3-nitrobenzolsulfonsäure-Na-Salz und
(B) 2-Chlor-1,4-phenylendiamin
Rotes Pulver, Schmelzpunkt über 250 °C

4. 2-Nitro-4'-dimethylaminodiphenylamin-4-sulfonsäure-Natriumsalz

Herstellung analog Beispiel 1 ausgehend von
(A) 4-Chlor-3-nitropbenzolsulfonsäure-Na-Salz und
(B) N,N-Dimethyl-p-phenylendiamin
Dunkelbraune Kristalle, Schmelzpunkt über 250 °C

5. 4-Nitro-4'-N,N-bis-(2-hydroxyethyl)-aminodiphenylamin-2-sulfonsäure-Natriumsalz

Herstellung analog Beispiel 1 ausgehend von
(A) 2-Chlor-5-nitrobenzolsulfonsäure-Na-Salz und
(B) N,N-Bis-(2-hydroxyethyl)-p-phenylendiamin
Rotes Pulver, D. C. Silikagel 60F-254 (Merck)
Laufmittel : n-Butanol/Essigsäure/Wasser = 8:2:1; Rf = 0,47

6. 4-Nitro-4'-dimethylaminodiphenylamin-2-sulfonsäure-Natriumsalz

Herstellung analog Beispiel 1 ausgehend von
(A) 2-Chlor-5-nitrobenzolsulfonsäure-Na-Salz und
(B) N,N-Dimethyl-p-phenylendiamin
Rotbraune Kristalle, Schmelzpunkt ca. 250 °C

7. 4-Nitro-3'-diemethylaminodiphenylamin-2-sulfonsäure

Herstullung nach Journ. Chem. Soc. (1948), 1229
Gelbbraune Kristalle Schmelzpunkt ca. 240 °C (unter Zersetzung)

Haarfärbeversuche

Es wurden Haarfärbecremes der folgenden Zusammensetzung hergestellt:

| | |
|---|---|
| Fettalkohol $C_{12-18}$ | 10 g |
| Fettalkohol $C_{12-14}$ + 2 EO-sulfat, Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiel 1 bis 7 eingesetzt.

Das Ergebnis der Färbeversuche ist der Tabelle I zu entnehmen.

Tabelle I

| direktziehender Farbstoff gemäß Beispiel | Nuance des gefärbten Haares |
|---|---|
| 1 | braun |
| 2 | rotblond |
| 3 | grauorange |
| 4 | braun |
| 5 | gelbbraun |
| 6 | gelb |
| 7 | tiefgelb |

## Patentansprüche

**1.** Verwendung von Verbindungen der Formel I

(I)

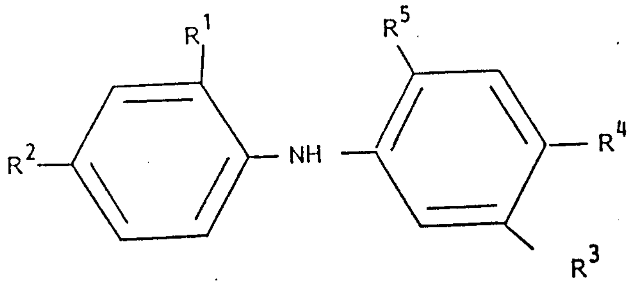

in der eine der Gruppen $R^1$ und $R^2$ eine Nitrogruppe und die andere eine $-SO_3H$-Gruppe und eine der Gruppen $R^3$ und $R^4$ eine Gruppe $-NR^6R^7$ ist, worin $R^6$ und $R^7$ Wasserstoff, Alkylgruppen mit 1 bis 4 C-Atomen oder Hydroxyalkylgruppen mit 2 bis 4 C-Atomen sind, und die andere sowie die Gruppe $R^5$ Wasserstoff, Chlor oder Alkylgruppen mit 1 bis 4 C-Atomen sind, oder deren wasserlösliche Salze als direktziehende Farbstoffe in Haarfärbemitteln.

**2.** Verwendung nach Anspruch 1, dadurch gekennzeichnet, daß $R^3$ Wasserstoff, $R^4$ eine Gruppe $-NR^6R^7$ und $R^5$ Wasserstoff, Chlor oder eine Methylgruppe ist.

**3.** Haarfärbemittel mit einem Gehalt an direktziehenden Farbstoffen in einem kosmetischen Träger, dadurch gekennzeichnet, daß als direktziehende Farbstoffe solche der Formel (I) gemäß Anspruch 1 oder 2 in einer Menge von 0,01 bis 5,0 Gew.-%, bezogen auf das gesamte Haarfärbemittel, enthalten sind.

**4.** Haarfärbemittel nach Anspruch 3, dadurch gekennzeichnet, daß zusätzlich zu den Verbindungen der Formel (I) noch weitere direktziehende Farbstoffe und/oder Oxidationsfarbstoffvorprodukte enthalten sind und daß diese Farbstoffe in einem kosmetischen Träger in Form einer Creme, einer Emulsion, eines Gels, eines Shampoos oder eines Schaumaerosols eingearbeitet sind.

## Claims

1. The use of compounds corresponding to formula I

(I)

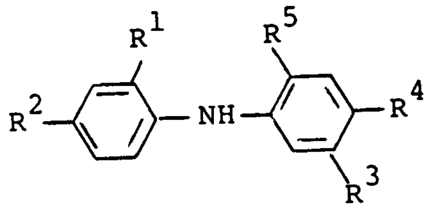

in which one of the groups $R^1$ and $R^2$ is a nitro group and the other an $-SO_3H$ group and one of the groups $R^3$ and $R^4$ is a group of the formula $-NR^6R^7$, where $R^6$ and $R^7$ are hydrogen, $C_{1-4}$ alkyl groups or $C_{2-4}$ hydroxyalkyl groups, while the other group and also the group $R^5$ are hydrogen, chlorine or $C_{1-4}$ alkyl groups,
or water-soluble salts of these compounds as substantive dyes in hair-dyeing preparations.

2. The use claimed in claim 1, characterized in that $R^3$ is hydrogen, $R^4$ is a group of the formula $-NR^6R^7$ and $R^5$ is hydrogen, chlorine or a methyl group.

3. Hair-dyeing preparations containing substantive dyes in a cosmetic carrier, characterized in that they contain as substantive dyes those corresponding to formula (I) according to claim 1 or 2 in a quantity of from 0.01 to 5.0% by weight, based on the hair-dyeing preparation as a whole.

4. Hair-dyeing preparations as claimed in claim 3, characterized in that, in addition to the compounds of formula (I), they contain other substantive hair dyes and/or oxidation dye precursors and in that these dyes are incorporated in a cosmetic carrier in the form of a cream, an emulsion, a gel, a shampoo or a foam aerosol.

## Revendications

1. Utilisation de composés de formule (I) ou de leurs sels hydrosolubles en tant que colorants à teinte directe dans les teintures pour les cheveux.

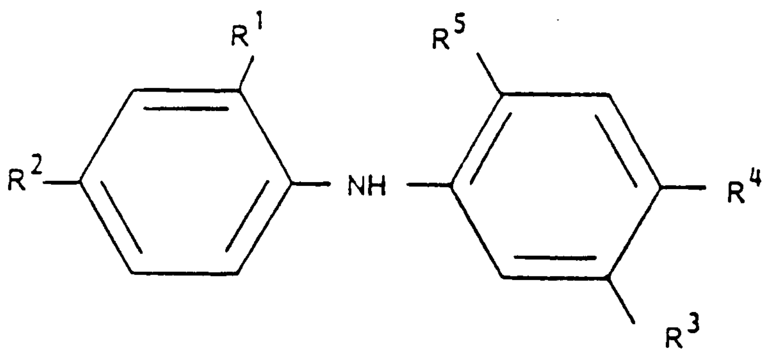

dans laquelle un des groupes $R_1$ et $R_2$ est un groupe Nitro et l'autre un groupe $SO_3H$, et dans laquelle un des groupes $R_3$ et $R_4$ est un groupe

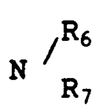

$R_6$ et $R_7$ représentant de l'hydrogène, des radicaux alcoyle avec 1 à 4 atomes de C ou des groupes hydroxyalcoyle ayant de 2 à 4 atomes de c et l'autre groupe ainsi que le groupe $R_5$ est de l'hydrogène, du chlore ou des radicaux alcoyle ayant de 1 à 4 atomes de C.

**2.** Utilisation selon la revendication 1, caractérisée en ce que $R_3$ est de l'hydrogène, $R_4$ est un groupe

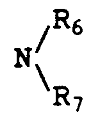

et $R_5$ est de l'hydrogène, du chlore ou un radical méthyle.

**3.** Teintures capillaires ayant un contenu en colorants à teinte directe dans un support cosmétique, caractérisées en ce que comme colorant à teinte directe, ceux de formule (I) selon la revendication 1 ou 2, y sont contenus en quantité allant de 0,01 à 5 % en poids rapporté à la teinture capillaire totale.

**4.** Teintures capillaires selon la revendication 3, caractérisées en ce qu'en plus des composés de formule (I), encore d'autres colorants à teinte directe et/ou des précurseurs de colorants par oxydation, y sont contenus et que ces colorants sont incorporés dans un support cosmétique sous forme d'une crème, d'une émulsion, d'un gel, d'un schampooing ou d'un aérosol de mousse.